# EUROPEAN PATENT SPECIFICATION

(11) **EP 4 149 414 B1**
(45) Date of publication and mention of the grant of the patent: **20.12.2023**
(21) Application number: 21739484.0
(22) Date of filing: 08.06.2021
(51) Int. Cl.: A61K 8/37, A61Q 1/12, A61K 8/02, A61K 8/92

(54) **COLORED COSMETIC SOLID DISPERSIONS**
GEFÄRBTE FESTE KOSMETISCHE DISPERSIONEN
DISPERSIONS SOLIDES COSMÉTIQUES COLORÉES

(30) Priority: 09.06.2020 US 202063036653 P; 15.04.2021 US 202163175122 P
(43) Date of publication of application: 22.03.2023
(73) Proprietor: Sun Chemical Corporation, Parsippany, NJ 07054 (US)
(72) Inventor: MADARAS, Mihaela, Cincinnati, OH 45232 (US); MICHIELS, Cindy, 1300 Wavre (BE); CLAPP, Lisa, Cincinnati, OH 45232 (US); CHAMBERS, Veronica, Cincinnati, OH 45232 (US); DEBACKER, Marguerite, 1340 Ottignies (BE)
(74) Representative: Carpmaels & Ransford LLP
(86) International application number: PCT/US2021/036303
(87) International publication number: WO 2021/252426

(56) References cited:
- US-A- 3 951 679
- US-A1- 2013 272 982
- US-A1- 2014 105 836
- US-A1- 2019 269 587

## Description

### BACKGROUND

There is a need in the cosmetics industry to use pigment preparations that are highly colored, low in pigment particle size, are easy to incorporate into various cosmetics formulations and are made with natural, plant-based ingredients. The present invention addresses all these requirements by providing a dry dispersion comprising three main components: (1) one or more D&C, FD&C organic or inorganic pigments or other pigments or colorants approved for direct contact cosmetic use in any jurisdiction ("pigments" in this application); (2) one or more naturally derived waxes with a melting temperature between 40-70⁰C; and (3) one or more naturally derived oils. The terms "natural", "naturally derived", "plant-based," and "bio-derived" may be used interchangeably throughout this application and all refer to materials that are derived naturally from renewable sources.
US 3,951,679 discloses blue colored mica flake pigments produced by coating mica flakes, or mica flakes optionally coated with a uniform metallic oxide layer, with a uniform layer of a low-solubility iron compound convertible into Berlin blue, and then converting this layer into Berlin blue. US 2013/272982 A1 discloses a method for imparting transfer resistance to a lipstick, comprising adding one or more colorants and a coconut alkane mixture, heating a coconut gel, and adding the colorant and coconut alkane mixture and coconut gel together. US 2014/105836 A1 discloses a lipstick including a colorant paste comprising one or more dry pigments and fractionated coconut oil, as well as a coconut gel. US 2019/269587 A1 discloses a spreadable conditioning composition comprising a blend of a branched hydrocarbon and an ester having a kinematic viscosity of less than or equal to about 100 centistokes.

### SUMMARY

Solid pigment dispersions and methods of making are described, wherein the dispersions are formulated to a paste consistency during grinding to reduce the pigment particle size and form a dry granular consistency at ambient temperature that is pourable and easy to handle. This allows for stronger, more intense colors and for an easier integration into a variety cosmetic products, cosmetic formulations, and personal care products. These pigment dispersions are prepared using plant-derived substances, obtained from naturally occurring, renewable sources. The solid pigment dispersion comprises one or more pigments approved for cosmetic use and a combination of one or more oils and one or more waxes that are plant-based, wherein the dispersion is in the form of a dry powder, wherein the pigment is present in an amount from 25-75%, by weight and the ratio of wax:oil is from 1:1 to 4:1, and wherein the particle size of the pigment is less than 15 µm as measured on a Hegman grind gauge according to the method specified in claim 1. The one or more oils and/or waxes may all be plant based.

The wax component of these solid pigment dispersions may be selected from the group consisting of rapeseed, soybean, sunflower, olive, castor bean, coconut, canola, jojoba, argan, pistachio, apricot kernel, sumac wax and combinations thereof. The oil component of these solid pigment dispersions may be selected from the group consisting of heptyl undecylenate, coco-caprylate, coco-caprylate/caprate, hydrogenated ethylhexyl olivate/hydrogenated olive oil unsaponifiables, propanediol dicaprylate, olive oil decyl esters/squalene and combinations thereof.

These solid pigment dispersions may have a melting point greater than 30°C and also greater than 50°C. These dispersions are also low-dusting, and the particle size of the pigment is less than 15 µm. The particle size of the pigment may be less than 10 µm.

### DETAILED DESCRIPTION

The process described herein is related to the preparation of a solid pigment dispersion by grinding a pigment to a particle size less than 15 µm, and also less than 10 µm, in order to develop stronger, more intense colors and to allow for easy integration into a variety cosmetic products. The dispersions are generally formulated to have a paste-like consistency during grinding in order to reduce the pigment particle size and to produce a dry granular consistency at ambient temperature that is pourable and easy to handle.

The advantages of these dry, solid dispersions are: (a) pigments in the dispersions are generally processed to a particle size less than 15 µm, and also less than 10 µm, regardless of the starting particle size; (b) the dispersions exhibit strong color due to the small particle size of the pigments and thus may be easily mixed into cosmetic products without additional processing; (c) the wax/oil base is plant-based, and not derived from unsustainable palm oil; and (d) the granular consistency at room temperature is low-dusting and allows for easy handling/pouring. In this application, "low-dusting" may be defined as dust travel distance in a graduated cylinder according to method described in paragraph [0058] and Table 30 to no more than 300 ml.

Additionally, other dispersants and additives may be added to the solid dispersion to impart additional properties such as color strength, compatibility with the intended application, functional properties, or physical properties such as the feel in a cosmetic.

The solid dispersions comprise high pigment content of 25-75%, for example 30-65%, by weight, combined with a natural wax and oil mix to produce a kneading consistency at an elevated temperature, for example 50-70°C, to allow for grinding of the pigment to a granular, dry, solid consistency when cooled to room temperature. Further dry milling of the dry, granular solid dispersion will provide a fine powder consistency that is easy to incorporate into compatible cosmetic formulations, such as for example lipstick, lip gloss, eye shadow, mascara and other similar products. In addition to cosmetics, the inventive solid dispersions are also suitable for a wide variety of personal care products, such as for example, shampoos, soaps, deodorants, etc. Though these solid dispersions are particularly suitable for direct skin contact products, they may also find uses in any application that typically incorporates pigment dispersions, such as for example inks, paints, colored films, textiles, paper and cellulosic applications and other industrial end uses.

One process to form these dispersions involves melting of a wax and oil mixture, typically at 60-70°C, followed by the addition of the pigment, generally at a concentration 50% or higher, by weight, to produce a paste of kneading consistency at 50-60°C. This paste may then be kneaded in an alpha blade mixer until the desired particle size of the pigment achieved, such as less than 15 µm, or also less than 10 µm. Once the desired particle size is obtained, the mixture is cooled, for example with dry ice, and broken down in the mixer to a granular consistency. Once cooled, the dispersion may be removed from the mixer. Depending on the desired granularity, the dispersion may be further processed in a Hammermill or similar dry milling equipment.

Pigments approved for cosmetic use in the present technology include but are not limited to black iron oxide (such as SunPuro or Suncroma Black Iron Oxide), red iron oxide (such as SunPuro or Suncroma Red Iron Oxide), brown iron oxide (such as SunPuro or Suncroma Brown Iron Oxide), yellow iron oxide (such as SunPuro or Suncroma Yellow Iron Oxide), titanium dioxide (such as SunCroma Titanium Dioxide), ultramarine blue (such as SunCroma Ultramarine Blue), manganese violet (such as SunCroma Manganese Violet), chromium oxide green (such as SunCroma Chromium Oxide Green), D&C Red 28 A1 lake (such as SunCroma D&C Red 28 A1 lake), D&C Red 22 A1 lake (such as SunCroma D&C Red 22 A1 lake), FD&C Blue 1 A1 lake (such as SunCroma, FD&C Blue 1 A1 lake), D&C Red 7 Ca lake (such as SunCroma D&C Red 7 Ca lake), D&C Red 6 Na salt (such as SunCroma D&C Red 6 Na salt), D&C Red 6 Ba lake (such as SunCroma D&C Red 6 Ba lake), FD&C Yellow 5 A1 lake (such as SunCroma FD&C Yellow 5 A1 lake), D&C Black 2 (such as SunCroma Black 2), other D&C and FD&C pigments that are approved for cosmetics use or meeting regulatory guidelines for cosmetics in other jurisdictions, and other pigments that are not specifically labeled as D&C or FD&C, but meet regulatory guidelines for cosmetic applications.

Ranges of pigment concentrations in the inventive compositions may be between 30-65%, by weight, and also between 40-60% by weight.

The naturally derived waxes included in this application may have a melting point above 30°C, or also above 50°C, and will produce a substance having a consistency which allows for pigment grinding to occur at 60-70°C and that solidifies at room temperature to produce low-dusting granular solid. The naturally derived waxes are derived from plant oils that undergo minimal transformations in order to stabilize against oxidation and increase the melting point.

Examples of such naturally derived waxes include, but are not limited to, hydrogenated oils from rapeseed, soybean, sunflower, olive, castor bean, coconut, canola, jojoba, argan, pistachio, apricot kernel, sumac (Rhus Verniciflua), in addition to the specific materials shown in Table 1.

**Table 1 - Examples of Commercially Available Naturally Derived Waxes**

| Wax type | Melting point (°C) |
|---|---|
| Hydrogenated rapeseed oil | 61-66 |
| Soybean (non -GMO) | 68-71 |
| Sunflower | 69-73 |
| Hydrogenated Rapeseed (low erucic) | 68 -74 |
| Hydrogenated Rapeseed (High erucic) | 61-66 |
| Hydrogenated Olive oil | 60-70 |
| Hydrogenated Castor bean oil | 86-88 |
| Hydrogenated Castor bean oil | >85 |
| Hydrogenated Coconut oil | 30-36 |
| Hydrogenated Canola oil | 70 |
| Hydrogenated Jojoba oil | 69 |
| Hydrogenated Argan Oil | 68 - 74 |
| Pistacia Vera Seed Oil | 55-65 |
| Apricot Kernel Oil | 67-73 |
| Rhus Verniciflua (Sumac) Peel Cera | 49 - 54 |

Examples of naturally derived, plant-based oils which may be used in the composition of this application include, but are not limited to, heptyl undecylenate, coco-caprylate, coco-caprylate/caprate, hydrogenated ethylhexyl olivate/hydrogenated olive oil unsaponifiables, propanediol dicaprylate, olive oil decyl esters/squalene, in addition to the specific materials shown in Table 2.

**Table 2 - Examples of Commercially Available Naturally Derived, Plant-Based Oils**

| Oil Type |
|---|
| Heptyl undecylenate |
| Glyceryl Undecylenate /Glyceryl Caprylate |
| Coco-Caprylate |
| C15-19 Alkane & Coco-Caprylate/Caprate |
| Hydrogenated Ethylhexyl Olivate, Hydrogenated Olive Oil Unsaponifiables |
| Propanediol Dicaprylate |
| Olive Oil Decylesters/Squalene |

The ratio of wax: oil in this application is from 1:1 to 4:1.

The inventive compositions of this application are ground to achieve a pigment particle size of less than 15 µm, and also less than 10 µm as measured on a Hegman grind gauge. Hegman method for particle size measurement was used according to ASTM D1210-05(2014) with additional heat. The method involves the application of the pre-melted dispersion on the Hegman gauge 50-0 microns channel, pre-heated to 50°C, using a scraper in order to obtain a continuous layer in the well from top to bottom. A good quality grind will show no scratches above 10 µm, indicating all the solid pigment particles are smaller than that.

The grinding of the inventive dispersions may be performed using any traditional pigment milling dispersing equipment known in the art, especially equipment designed to handle high viscosity materials. Example of such equipment includes as sigma blade mixers, three roll mills and kneaders.

Existing cosmetic dispersions are either liquid/viscous, paste/gel or paste that often require a viscosity measurement and the use of suspending agents to keep the pigment suspended in the dispersion and prevent settling. The inventive dispersions of the present application are a dry solid at room temperature and may be ground into flowable powders. It is also well known to those skilled in the art that the pourability and ease of handling of flowable powders is much preferred to the handling of thick pastes. The dispersions of the present invention do not require suspending agent nor viscosity measurement, since pigment settling does not occur, providing stable dispersions of pigment within the dispersing matrix.

Further, liquid, paste and gel dispersions often require premixing prior to their use which is not necessary with the dry solid dispersions of the present invention. Color consistency problems associated with liquid, paste and gel dispersions are not an issue with the dispersions of the present invention since settling does not occur and premixing is not necessary.

Compared to traditional pigment powders, the dispersions of the present invention can be used without a decreased risk of dusting during handling, as they have the flow characteristics of a dry powder pigment with the advantage of having the pigment fully & homogeneously dispersed /grinded/embedded into a solid matrix, greatly decreasing the dusting characteristics (see Table 30 below).

In addition, the inventive dispersions produce less dust than pigments, making them safer to use in an industrial environment when compared to the starting pigments (see dusting data and test method below).

The inventive dispersions offer improvements for cosmetic applications including but not limited to: lipstick, concealer stick, eyeliner, lip balm, blusher cream, matte lip cream, foundation, cream-to-powder concealer, cream-to-powder blush, cream-to-powder lip color, cream-to-powder eye shadow, Water/Oil emulsions such as those used in BB Cream (Blemish Balm Cream) and in CC Cream (Color Correcting Cream), and oil/water emulsions such as face primers.

The present invention has been described in detail, including the preferred embodiments thereof. However, it will be appreciated that those skilled in the art, upon consideration of the present disclosure, may make modifications and/or improvements on this invention that fall within the scope of the invention as defined by the appended claims.

### EXAMPLES

The technology of this application may be further described by the following non-limiting examples which further illustrate the technology, and are not intended, nor should they be interpreted to, limit the scope. Percentages of materials are as percent-by-weight.

**Table 3: examples of the inventive dispersion compositions and of waxes and oil options. (% by weight)**

| Ex. # | Pigment | Pigment Code | Pigment % | Oil | Oil % | Wax | Wax % |
|---|---|---|---|---|---|---|---|
| R4223-59 (Ex. 1) | Suncroma FD&C Yellow 5 Al Lake | C69-45 3 7 | 50 | Heptyl undecylenate | 14.6 | Hydrogenated rapeseed oil | 35.4 |
| R3980-153 (Ex. 2) | Suncroma D&C Red 6 Na salt | C19-6619 | 50 | Heptyl undecylenate | 14.6 | Hydrogenated rapeseed oil | 35.4 |
| R4223-55 (Ex. 3) | Soft-Tex D&C Red 6 Ba Lake | C19-7712 | 50 | Heptyl undecylenate | 14.6 | Hydrogenated rapeseed oil | 35.4 |
| R4223-28 (Ex. 4) | Soft-Tex D&C Red 7 Ca Lake | C19-7711 | 50 | Heptyl undecylenate | 14.6 | Hydrogenated rapeseed oil | 35.4 |
| R4223-58 (Ex. 5) | Suncroma FD&C Blue 1 Al lake | C39-4433 | 50 | Heptyl undecylenate | 14.6 | Hydrogenated rapeseed oil | 35.4 |
| R4223-27 (Ex. 6) | Suncroma D&C Red 28 Al Lake | C14-6623 | 50 | Heptyl undecylenate | 14.6 | Hydrogenated rapeseed oil | 35.4 |
| R4223-53 (Ex. 7) | SunPuro Yellow Iron Oxide | C33-9001 | 50 | Heptyl undecvlenate | 14.6 | Hydrogenated rapeseed oil | 35.4 |
| R4223-52 (Ex. 8) | SunPuro Red Iron Oxide | C33-8001 | 50 | Heptyl undecvlenate | 14.6 | Hydrogenated rapeseed oil | 35.4 |
| R4223-54 (Ex. 9) | SunPuro Black Iron Oxide | C33-7001 | 50 | Heptyl undecylenate | 14.6 | Hydrogenated rapeseed oil | 35.4 |
| R4223-103 (Ex. 10) | Soft-Tex D&C Red 7 Ca Lake | C19-7711 | 50 | C15-19 Alkane (& Coco-Caprylate/Caprate) | 14.6 | Natural Pistachio Wax | 35.4 |
| R4223-104 (Ex. 11) | Soft-Tex D&C Red 7 Ca Lake | C19-7711 | 50 | Triheptanoin (and) C13-15 Alkane | 14.6 | Hydrogenated Canola Oil | 35.4 |
| R4223-105 (Ex. 12) | Soft-Tex D&C Red 7 Ca Lake | C19-7711 | 50 | Hydrogenated Ethylhexyl Olivate, Hydrogenated Olive Oil Unsaponifiables | 14.6 | Hydrogenated Castor Oil | 35.4 |
| R4223-106 (Ex. 13) | Soft-Tex D&C Red 7 Ca Lake | C19-7711 | 50 | Heptyl undecylenate | 20 | Hydrogenated rapeseed oil | 30 |
| R4223-107 (Ex. 14) | Soft-Tex D&C Red 7 Ca Lake | C19-7711 | 50 | Heptyl undecylenate | 10 | Hydrogenated rapeseed oil | 40 |
| R4223-108 (Ex. 15) | Soft-Tex D&C Red 7 Ca Lake | C19-7711 | 40 | Heptyl undecylenate | 17.5 | Hydrogenated rapeseed oil | 42.5 |
| R4223-109 (Ex. 16) | Soft-Tex D&C Red 7 Ca Lake | C19-7711 | 65 | Heptyl undecylenate | 14 | Hydrogenated rapeseed oil | 21 |
| R4223-18 (Ex. 17) | Suncroma Titanium Dioxide | C47-A053 | 50 | Heptyl undecylenate | 14.6 | Hydrogenated rapeseed oil | 35.4 |
| R4223-29 (Ex. 18) | Suncroma Ultramarine Blue | C43-1810 | 50 | Heptyl undecylenate | 14.6 | Hydrogenated rapeseed oil | 35.4 |
| R4223-30 (Ex. 19) | Suncroma Chromium Oxide | C61-1245 | 50 | Heptyl undecylenate | 14.6 | Hydrogenated rapeseed oil | 35.4 |
| R4223-31 (Ex. 20) | Suncroma Manganese Violet | C43-001 | 50 | Heptyl undecylenate | 14.6 | Hydrogenated rapeseed oil | 35.4 |
| EXP1850A-032 (Ex. 21) | Suncroma D&C Red 7 Ca Lake | C19-003 | 32 | Heptyl undecylenate | 20 | Hydrogenated rapeseed oil | 48 |
| EXP1850A-034 (Ex. 22) | Suncroma D&C Red 7 Ca Lake | C19-003 | 34 | Heptyl undecylenate | 18 | Hydrogenated rapeseed oil | 48 |
| EXP4510-032 (Ex. 23) | Suncroma D&C Red 28 Al Lake | C14-6623 | 32 | Heptyl undecylenate | 20 | Hydrogenated rapeseed oil | 48 |
| EXP4510-034 (Ex. 24) | Suncroma D&C Red 28 Al Lake | C14-6623 | 34 | Heptyl undecylenate | 18 | Hydrogenated rapeseed oil | 48 |
| EXP77492-032 (Ex. 25) | Suncroma Yellow Iron Oxide | C33-A8073 | 32 | Heptyl undecylenate | 20 | Hydrogenated rapeseed oil | 48 |
| EXP77492-034 (Ex. 26) | Suncroma Yellow Iron Oxide | C33-A8073 | 34 | Heptyl undecylenate | 18 | Hydrogenated rapeseed oil | 48 |
| EXP77491-032 (Ex. 27) | Suncroma Red Iron Oxide | C33-A8075 | 32 | Heptyl undecvlenate | 20 | Hydrogenated rapeseed oil | 48 |
| EXP77491-034 (Ex. 28) | Suncroma Red Iron Oxide | C33-A8075 | 34 | Heptyl undecylenate | 18 | Hydrogenated rapeseed oil | 48 |
| EXP77499-032 (Ex. 29) | Suncroma Black Iron Oxide | C33-A5198 | 32 | Heptyl undecvlenate | 20 | Hydrogenated rapeseed oil | 48 |
| EXP77499-034 (Ex. 30) | Suncroma Black Iron Oxide | C33-A5198 | 34 | Heptyl undecylenate | 18 | Hydrogenated rapeseed oil | 48 |
| EXP42090-034 (Ex. 31) | Suncroma FD&C Blue 1 Al lake | C39-4433 | 32 | Heptyl undecvlenate | 20 | Hydrogenated rapeseed oil | 48 |
| EXP42090-034 (Ex. 32) | Suncroma FD&C Blue 1 Al lake | C39-4433 | 34 | Heptyl undecylenate | 18 | Hydrogenated rapeseed oil | 48 |
| EXP77891-032 (Ex. 33) | Suncroma Titanium Dioxide | C47-056 | 32 | Heptyl undecylenate | 20 | Hydrogenated rapeseed oil | 48 |
| EXP77891-034 (Ex. 34) | Suncroma Titanium Dioxide | C47-056 | 34 | Heptyl undecylenate | 18 | Hydrogenated rapeseed oil | 48 |
| EXP1850B-032 (Ex. 35) | Suncroma D&C Red 6 Na salt | C19-6619 | 32 | Heptyl undecylenate | 20 | Hydrogenated rapeseed oil | 48 |
| EXP1850B-034 (Ex. 36) | Suncroma D&C Red 6 Na salt | C19-6619 | 34 | Heptyl undecylenate | 18 | Hydrogenated rapeseed oil | 48 |

All grind readings for Examples 1-36 were ≤ 10µm as measured on a Hegman grind gauge.

The inventive solid dispersions were incorporated into a series of various cosmetic formulations demonstrating their suitability for these end-use applications. The solid dispersion formulations are non-limiting, and it is clear that the inventive dispersions may be used in other personal care and cosmetic applications (e.g. soaps, shampoos, etc.)

**Table 4 - Natural Base Lipstick (Example 37) (% by weight)**

| Phase | Ingredients | INCI | % | Suppliers |
|---|---|---|---|---|
| A | Sweet Almond Oil | Prunus Amygdalus Dulcis (Sweet Almond Oil) | 50.63 | Jand Dekker /IMCD |
| | Melon Seed Oil Refined | Citrullus Vulgaris Seed Oil | 15.75 | African Origin Oils/DKSH |
| | Kahlwax 2039L | Euphorbia Cerifera Cera | 12.00 | Kahlwax |
| | Olive Butter SP-515 | - | 7.00 | STRAHL & PITSCH LLC |
| | Kahlwax 6279L | Myrica Cerifera Fruit Wax | 4.00 | Kahlwax |
| | Kahlwax 8104 | Cera Alba | 4.00 | Kahlwax |
| | Natural Wax Jelly SP-511 | - | 2.50 | STRAHL & PITSCH LLC |
| | | | | |
| B | R4223-27 (Ex. 6) | D&C Red 28 Al Lake Natural Wax Dispersion (SunChemical) | 20.00 | SunChemical |
| | | | | |
| C | Geogard 111 A | Dehydroacetic Acid | 0.30 | Lonza/Azelis |
| Procedure | 1. Heat phase A ingredients together until molten | | | |
| | 2. Mix and melt phase B ingredients until uniform | | | |
| | 3. Add phase B to A and mix until homogeneous | | | |
| | 4. Add phase C and mix until homogeneous | | | |
| | 5. Pour into mold, then allow to cool | | | |

**Table 5 - Natural base - Concealer Stick Medium shade (Example 38) (% by weight)**

| Phase | Ingredients | INCI | % | Suppliers |
|---|---|---|---|---|
| A | Almond Oil | Prunus Amygdalus Dulcis (Sweet Almond) Oil | 35.51 | Jand Dekker /IMCD |
| | Melon Seed Oil Refined | Citrullus Vulgaris Seed Oil | 11.98 | African Origin Oils/DKSH |
| | Kahlwax 2039L | Euphorbia Cerifera Cera | 10.27 | Kahlwax |
| | Olive Butter SP-515 | - | 6.00 | STRAHL & PITSCH LLC |
| | Kahlwax 6279L | Myrica Cerifera Fruit Wax | 3.42 | Kahlwax |
| | Kahlwax 8104 | Cera Alba | 3.42 | Kahlwax |
| | Natural Wax Jelly SP-511 | - | 2.10 | STRAHL & PITSCH LLC |
| | | | | |
| B | R4223-54 (Ex. 9) | Black Iron Oxide Natural Wax Dispersion | 0.34 | SunChemical |
| | R4223-53 (Ex. 7) | Yellow Iron Oxide Natural Wax Dispersion | 2.37 | SunChemical |
| | R4223-52 (Ex. 8) | Red Iron Oxide Natural Wax Dispersion | 1.01 | SunChemical |
| | R4223-18 (Ex. 17) | Titanium Dioxide Natural Wax Dispersion | 20.28 | SunChemical |
| | | | | |
| C | Geogard 111 A | Dehydroacetic Acid | 0.30 | Lonza/Azelis |
| | | | | |
| D | C86-6105 Satin Mica | Mica | 3.00 | SunChemical |
| Procedure | 1. Heat phase A ingredients together until molten | | | |
| | 2. Mix and melt phase B ingredients until uniform | | | |
| | 3. Add phase B to A and mix until homogeneous | | | |
| | 4. Add phase C and mix until homogeneous | | | |
| | 4. Add phase D and mix until homogeneous | | | |
| | 6. Pour into mold, then allow to cool | | | |

**Table 6 - Natural base - Concealer Stick Dark shade (Example 39) (% by weight)**

| Phase | Ingredients | INCI | % | Suppliers |
|---|---|---|---|---|
| A | Almond Oil | Prunus Amygdalus Dulcis (Sweet Almond Oil) | 35.51 | Jand Dekker /IMCD |
| | Melon Seed Oil Refined | Citrullus Vulgaris Seed Oil | 11.98 | African Origin Oils/DKSH |
| | Kahlwax 2039L | Euphorbia Cerifera Cera | 10.27 | Kahlwax |
| | Olive Butter SP-515 | - | 6.00 | STRAHL & PITSCH LLC |
| | Kahlwax 6279L | Myrica Cerifera Fruit Wax | 3.42 | Kahlwax |
| | Kahlwax 8104 | Cera Alba | 3.42 | Kahlwax |
| | Natural Wax Jelly SP-511 | - | 2.10 | STRAHL & PITSCH LLC |
| | | | | |
| B | R4223-54 (Ex. 9) | Black Iron Oxide Natural Wax Dispersion | 6.56 | SunChemical |
| | R4223-53 (Ex. 7) | Yellow Iron Oxide Natural Wax Dispersion | 8.72 | SunChemical |
| | R4223-52 (Ex. 8) | Red Iron Oxide Natural Wax Dispersion | 8.72 | SunChemical |
| | | | | |
| C Geogard 111 A | | Dehydroacetic Acid | 0.30 | Lonza/Azelis |
| | | | | |
| D | C86-6105 Satin Mica | Mica | 3.00 | SunChemical |
| Procedure | 1. Heat phase A ingredients together until molten | | | |
| | 2. Mix and melt phase B ingredients until uniform | | | |
| | 3. Add phase B to A and mix until homogeneous | | | |
| | 4. Add phase C and mix until homogeneous | | | |
| | 4. Add phase D and mix until homogeneous | | | |
| | 6. Pour into mold, then allow to cool | | | |

**Table 7 - Natural Base - Cake Eye Liner (Example 40) (% by weight)**

| Phase | Ingredients | INCI | % | Suppliers |
|---|---|---|---|---|
| A | Almond Oil | Prunus Amygdalus Dulcis (Sweet Almond) Oil | 51.19 | Jand Dekker /IMCD |
| | Melon Seed Oil Refined | Citrullus Vulgaris Seed Oil | 15.80 | African Origin Oils/DKSH |
| | Kahlwax 6279L | Myrica Cerifera Fruit Wax | 13.50 | Kahlwax |
| | Olive Butter SP-515 | - | 7.90 | STRAHL & PITSCH LLC |
| | Kahlwax 2039L | Euphorbia Cerifera Cera | 4.50 | Kahlwax |
| | Kahlwax 8104 | Cera Alba | 4.50 | Kahlwax |
| | Natural Wax Jelly SP-511 | - | 2.25 | STRAHL & PITSCH LLC |
| | | | | |
| B | Geogard 111 A | Dehydroacetic Acid | 0.36 | Lonza/Azelis |
| Color Components | | | | |
| C | R4223-54 (Ex. 9) | Black Iron Oxide Natural Wax Dispersion | 30.00 | SunChemical |
| Procedure | 1. Heat phase A ingredients together until molten | | | |
| | 2. Mix and melt phase B ingredient until uniform | | | |
| | 3. Add phase B to A and mix until homogeneous | | | |
| | 4. Add phase C and mix until homogeneous | | | |
| | 5. Pour into mold, then allow to cool | | | |

**Table 8 - Natural Base - Lip Balm (Example 41) (% by weight)**

| Phase | Ingredients | INCI | % | Suppliers |
|---|---|---|---|---|
| A | Kahlbase 7704 | Helianthus Annuus Seed Oil, Simmondsia Chinensis Seed Oil, Ricinus Communis Seed Oil, Rhus Verniciflua Peel Cera, Cera Alba, Helianthus Annuus Seed Cera, Euphorbia Cerifera Cera, Shorea Robusta Resin, Tocopherol, Ascorbyl Palmitate | 89.0 | KahlWax |
| | R4223-18 (Ex. 17) | Titanium Dioxide Natural Wax Dispersion | 1.00 | SunChemical |
| | C92-4210 SunSHINE^{®} Golden Sunrise | Synthetic Fluorphlogopite (and) Titanium Dioxide (and) Magnesium Silicate | 9.50 | SunChemical |
| | C91-422 1INTENZA^{®} Orange Zest | Synthetic Fluorphlogopite (and) Titanium Dioxide (and) D&C Red 6 Sodium Salt | 0.50 | SunChemical |

**Table 9 - Natural Base - Lip Paint (Example 42) (% by weight)**

| Phase | Ingredients | INCI | % | Suppliers |
|---|---|---|---|---|
| A | Almond Oil | Prunus Amygdalus Dulcis (Sweet Almond) Oil | 51.19 | Jand Dekker /IMCD |
| | Melon Seed Oil Refined | Citrullus Vulgaris Seed Oil | 15.80 | African Origin Oils/DKSH |
| | Kahlwax 6279L | Myrica Cerifera Fruit Wax | 13.50 | Kahlwax |
| | Olive Butter SP-515 | - | 7.90 | STRAHL & PITSCH LLC |
| | Kahlwax 2039L | Euphorbia Cerifera Cera | 4.50 | Kahlwax |
| | Kahlwax 8104 | Cera Alba | 4.50 | Kahlwax |
| | Natural Wax Jelly SP-511 | - | 2.25 | STRAHL & PITSCH LLC |
| | | | | |
| B | Geogard 111 A | Dehydroacetic Acid | \| 0.36 | \| Lonza/Azelis |

| Color Components | | | | |
|---|---|---|---|---|
| C | R4223-28 (Ex. 4) | D&C Red 7 Ca Lake Natural Wax Dispersion | 10.00 | SunChemical |
| | R3980-153 (Ex. 2) | D&C Red 6 Na Salt Natural Wax Dispersion | 3.00 | SunChemical |
| | C84-4215 SunSHINE^{®} Spectral Golden | Synthetic Fluorphlogopite (and) Titanium Dioxide (and) Iron Oxide | 1.00 | SunChemical |
| | C86-3111 SynMICA Fine | Synthetic Fluorphlogopite | 3.00 | SunChemical |
| D | R4223-59 (Ex. 1) | FD&C Yellow 5 Al Lake Natural Wax Dispersion | 30.00 | SunChemical |
| | C84-6125 SunSHINE^{®} Spectral Orange | Synthetic Fluorphlogopite (and) Iron Oxide | 5.00 | SunChemical |
| Procedure | 1. Heat phase A ingredients together until molten | | | |
| | 2. Mix and melt C until uniform | | | |
| | 3. Add C to phase A and mix until homogeneous | | | |
| | 4. Add phase B and mix until homogeneous | | | |
| | 5. Add D and mix until homogeneous | | | |
| | 6. Pour into mold, then allow to cool | | | |

**Table 10 - Natural Base - Blusher Cream (Example 43) (% by weight)**

| Phase | Ingredients | INCI | % | Suppliers |
|---|---|---|---|---|
| A | Almond Oil | Prunus Amygdalus Dulcis (Sweet Almond) Oil | 51.19 | Jand Dekker /IMCD |
| | Melon Seed Oil Refined | Citrullus Vulgaris Seed Oil | 15.80 | African Origin Oils/DKSH |
| | Kahlwax 6279L | Myrica Cerifera Fruit Wax | 13.50 | Kahlwax |
| | Olive Butter SP-515 | - | 7.90 | STRAHL & PITSCH LLC |
| | Kahlwax 2039L | Euphorbia Cerifera Cera | 4.50 | Kahlwax |
| | Kahlwax 8104 | Cera Alba | 4.50 | Kahlwax |
| | Natural Wax Jelly SP-511 | - | 2.25 | STRAHL & PITSCH LLC |
| | | | | |
| B | Geogard 111 A | Dehydroacetic Acid | 0.36 | Lonza/Azelis |

| Color Components | | | | |
|---|---|---|---|---|
| C | R4223-28 (Ex. 4) | D&C Red 7 Ca Lake Natural Wax Dispersion | 18.00 | SunChemical |
| | R4223-58 (Ex. 5) | FD&C Red Blue 1 Al Lake Natural Wax Dispersion | 6.00 | SunChemical |
| D | C84-4215 SunSHINE^{®} Spectral Golden | Synthetic Fluorphlogopite (and) Titanium Dioxide (and) Iron Oxide | 1.00 | SunChemical |
| | C86-3111 SynMICA Fine | Synthetic Fluorphlogopite | 3.00 | SunChemical |
| Procedure | 1. Heat phase A ingredient together until molten | | | |
| | 2. Mix and melt C until uniform | | | |
| | 3. Add C to phase A and mix until homogeneous | | | |
| | 4. Add phase B and mix until homogeneous | | | |
| | 5. Add D and mix until homogeneous | | | |
| | 6. Pour into mold, then allow to cool | | | |

**Table 11 - Long Lasting Matte Lip Cream (Example 44) (% by weight)**

| Phase | Ingredient | INCI | % | Supplier |
|---|---|---|---|---|
| A | Arlamol HD | Isohexadecane | 48.6 0 | IMCD |
| | Bentone Gel ISD V | Isododecane, Disteardimonium, Hectorite, Propylene Carbonate | 12.8 0 | Elementis |
| | CRODAMOL PTIS-LQ-(MV) | Pentaerythrityl Tetraisostearate | 8.75 | Croda |
| | | | | |
| B | Kahlwax 1540 - Beeswax Substitute | Cera Microcristallina, Hydrogenated Vegetable Oil, Cera Alba, Hydrogenated Palm Acid, Stearyl Stearate | 5.80 | KahlWax |
| | Xiameter PMX-200 Silicone Fluid 5cs | Polydimethylsiloxane Polymer | 5.80 | Dow |
| | Compritol 888CG | Tribehenin | 4.85 | Gattefossé |
| | KSG-19 | Dimethicone, Dimethicone/Vinyl Dimethicone Crosspolymer | 3.90 | Shin Etsu |
| | Lexgard O | Caprylyl Glycol | 0.95 | Inolex |
| | | | | |
| C | CL-Silica-150 | Silica | 3.85 | Chemland/Maprec os |
| | SynMica C86-3222 | Synthetic Fluorphlogopite | 4.90 | SunChemical |
| | | | | |

| Color Components | | | | |
|---|---|---|---|---|
| D | R3980-153 (Ex. 2) | D&C Red 6 Na Salt Natural Wax Dispersion | 20.0 0 | SunChemical |
| | COD-8003 | D&C Red 6 Na Salt Castor Oil Dispersion | 20.0 0 | SunChemical |
| | SWD-4519 | D&C Red 6 Na Salt Synthetic Wax Dispersion | 20.0 0 | SunChemical |
| Procedur e | 1. Homogenize Phase A ingredients for 15 minutes | | | |
| | 2. Add ingredients of Phase B to Phase A, then heat to 80°C | | | |
| | 3. Add ingredient of Phase C under stirring and allow it to cool | | | |
| | 4. Premix ingredients D, then add to the A, B, C mixture | | | |
| | 5. Fill into suitable packaging | | | |

**Table 12 - CREAMY MASCARA with Colored Dry Dispersion (Example 45) (% by weight)**

| Phase | Ingredients | INCI | Supplier | % |
|---|---|---|---|---|
| A | Stearic Acid 98% | Stearic Acid | Mosselman | 12 |
| | Nikkol IOP | Ethylhexyl Palmitate | Nikkol | 7.6 |
| | Cerasynt 945 | Glyceryl Stearate, Laureth-23 | ISP | 4.7 |
| | S&M Propylparaben | Propylparaben | Schiilke & Mayr | 0.1 |
| | | | | |
| B | Distilled Water | Aqua | Local | 52.2 |
| | Propanediol | Propylene Glycol | Acros / Fisher | 9.5 |
| | Triethanolamine 99% | Triethanolamine | Mosselman | 3.8 |
| | S&M Methylparaben | Methylparaben | Schülke & Mayr | 0.1 |

| Color Components | | | | |
|---|---|---|---|---|
| C | R4223-54 (Ex. 9) | Black Iron Oxide Dry Dispersion | SunChemical | 20 |
| Procedures | | | | |
| Procedure 1: Add Dry Dispersion before emulsion | 1. Heat separately NWD & Phase A to 70 C° then mix them together | | | |
| | 2. Heat Phase B to 70 C° | | | |
| | 3. When the Colored Dry dispersion is completely melted and homogenously dispersed, add Phase B while maintaining high speed mixing. | | | |
| | 4. Maintain low agitation until the emulsion reaches 40C°. | | | |
| | 5. Fill into suitable packaging | | | |
| Procedure 2: Add Dry Dispersion after emulsion | 1. Heat Phase A and Phase B to 70 C°. | | | |
| | 2. Add Phase A to Phase B maintaining high stirring. | | | |
| | 3. Melt dry dispersion to 70 C°, then add it to the mix; homogenize and maintain low stirring until 40 C°. | | | |
| | 4. Fill into suitable packaging | | | |

**Table 13 - GLOSSY LIP GLOSS (Example 46) (% by weight)**

| Ingredients | INCI | % | Supplier |
|---|---|---|---|
| Indopol H-300 | Polybutene | 41.9 | Ineos Oligomers |
| Crodamol PTIS | Pentaerythrityl Tetraisostearate | 15.6 | Croda |
| Octyldodecanol | Octyldodecanol | 15.6 | KahlWax |
| Liponate TDTM | Tridecyl Trimellitate | 10.5 | Lipo France |
| Silkflo 366 NF | Hydrogenated Polydecene | 2.1 | Lipo France |
| Super Sterol Ester | C10-C30 Cholesterol, Lanosterol Esters | 9.5 | Croda |
| Beeswax White EP | Cera Alba | 2.3 | Mosselman |
| S&M Methylparaben | Methylparaben | 0.3 | Schulke & Mayr |
| S&M Propylparaben | Propylparaben | 0.2 | Schulke & Mayr |
| Oxynex K | PEG-8, Tocopherol, Ascorbyl Palmitate, Ascorbic Acid, Citric Acid | 0.2 | Merck |
| Vitamin E Acetate USP | Tocopheryl Acetate | 0.3 | BASF |
| Hydrogenated Castor Oil | Hydrogenated Castor Oil | 1.5 | Mosselman |
| Bilberry | Aroma | 5 drops | Ipra France |

| Color Components | | | |
|---|---|---|---|
| R4223-28 (Ex. 4) | D&C Red 7 Ca Lake Dry Dispersion | 20 | SunChemical |
| COD-8001 | D&C Red 7 Ca Lake Castor Oil Dispersion | 20 | SunChemical |
| SWD-4511 | D&C Red 7 Ca Lake Synthetic Wax Dispersion | 20 | SunChemical |
| Procedure | 1. Pre-melt mixed color components | | |
| | 2. Weigh all ingredients of the base and heat to ∼80 C°, mix well until they are all melted and homogenous | | |
| | 3. Add in desired color dry dispersion mix and homogenize. | | |
| | 4. Fill into suitable packaging | | |

**Table 14 - Cream-to-Powder Foundation (Example 47) (% by weight)**

| Ingredients | INCI | Light shade, % | Dark shade, % |
|---|---|---|---|
| Dry dispersion mix | Color composition | 15 | 15 |
| Dub 810 C | Cococaprylate/caprate | 25 | 30 |
| Premium Organic Olive Butter | - | 10 | 10 |
| Calcium Carbonate | Calcium carbonate | 20 | 20 |
| Satin Mica C86 | 6105 Mica | 20 | 20 |
| Sepifine BB | Amylopectin | 5 | 5 |
| Lipex Shealight | Shea butter ethyl esters | 5 | - |

**Table 15 - Cream-to-Powder Concealer (Example 48) (% by weight)**

| Ingredients | INCI | Light shade, % |
|---|---|---|
| Dry dispersion mix | Color composition | 15 |
| Dub 810 C | Cococaprylate/caprate | 25 |
| Premium Organic Olive Butter | - | 10 |
| Lipex Shealight | Shea butter ethyl esters | 20 |
| Calcium Carbonate | Calcium carbonate | 12.5 |
| Satin Mica C86 | 6105 Mica | 12.5 |
| Sepifine BB | Amylopectin | 5 |

**Table 16: Color compositions for Cream-to-Powder Foundation and Concealer (Example 49) (% by weight)**

| | Dry dispersion | INCI | % |
|---|---|---|---|
| Light shade | R4223-18 (Ex. 17) | Suncroma Titanium Dioxide | 12.7 |
| | R4223-52 (Ex. 8) | SunPuro Red Iron Oxide | 0.6 |
| | R4223-53 (Ex. 7) | SunPuro Yellow Iron Oxide | 1.5 |
| | R4223-54 (Ex. 9) | SunPuro Black Iron Oxide | 0.2 |
| Dark shade | R4223-54 (Ex. 9) | SunPuro Black Iron Oxide | 4.1 |
| | R4223-53 (Ex. 7) | SunPuro Yellow Iron Oxide | 5.45 |
| | R4223-52 (Ex. 8) | SunPuro Red Iron Oxide | 5.45 |

**Table 17 - 2-in-1 Cream-to-Powder Pearly Blush and Lip Color (Example 50) (% by weight)**

| Ingredients | INCI | Blush, % | Lip color, % |
|---|---|---|---|
| R4223-27 (Ex. 6) | Suncroma D&C Red 28 Al Lake | 10 | 8 |
| Dub 810 C | Cococaprylate/caprate | 25 | 27 |
| Premium Organic Olive Butter | - | 10 | 10 |
| C98-A790M Suncroma Methicone treated Sericite | Mica & Methicone | 23 | 23 |
| C84-6288 Sunpuro Bronze | Mica & Red Iron Oxide | 7 | 7 |
| C81-1218 Sunpuro Iridescent Gold | Mica and Titanium Dioxide | 20 | 20 |
| Sepifine BB | Amylopectin | 5 | 5 |
| Isodecane | Isodecane | few drops | 3 |

**Table 18 - Cream-to-Powder Blue Eye Shadow (Example 51) (% by weight)**

| Ingredients | INCI | Blue, % |
|---|---|---|
| R4223-29 (Ex. 18) | Suncroma Ultramarine Blue | 15 |
| Dub 810 C | Cococaprylate/caprate | 25 |
| Premium Organic Olive Butter | - | 10 |
| Calcium Carbonate | Calcium carbonate | 20 |
| Satin Mica C86-6105 | Mica | 15 |
| C83-1254 SunPuro Deep Blue | Mica & TiO2 & Iron Blue | 5 |
| Sepifine BB | Amylopectin | 5 |
| Lipex Shealight | Shea butter ethyl esters | 5 |

**Table 19 - Cream-to-Powder Green Eye Shadow (Example 52) (% by weight)**

| Ingredients | INCI | Light Green, % |
|---|---|---|
| R4223-30 (Ex. 19) | Suncroma Chromium Oxide | 10 |
| R4223-18 (Ex. 17) | Suncroma Titanium Dioxide | 5 |
| Dub 810 C | Cococaprylate/caprate | 25 |
| Premium Organic Olive Butter | - | 10 |
| Calcium Carbonate | Calcium carbonate | 20 |
| Satin Mica C86-6105 | Mica | 15 |
| C91-4161 Intenza Envy | FD&C Blue 1 Al Lake & FD&C Yellow 5 Al Lake | 5 |
| C80-3100 SunSHINE Fine White | Synthetic Fluorphlogite and Titanium dioxide | |
| Sepifine BB | Amylopectin | 5 |
| Lipex Shealight | Shea butter ethyl esters | 5 |

**Table 20 - Cream-to-Powder Violet Eye Shadow (Example 53) (% by weight)**

| Ingredients | INCI | Purple, % |
|---|---|---|
| R4223-31 (Ex. 20) | Suncroma Manganese Violet | 10 |
| R4223-18 (Ex. 17) | Suncroma Titanium Dioxide | 5 |
| Dub 810 C | Cococaprylate/caprate | 25 |
| Premium Organic Olive Butter | - | 10 |
| Calcium Carbonate | Calcium carbonate | 20 |
| Satin Mica C86-6105 | Mica | 15 |
| C81-3740 SunSHINE Super glitter Violet | Synthetic Fluorphlogite and Titanium dioxide | 5 |
| Sepifine BB | Amylopectin | 5 |
| Lipex Shealight | Shea butter ethyl esters | 5 |

**Table 21- 2-in-1 Cream-to-Powder Blue Pearly Eye Shadow (Example 54) (% by weight)**

| Ingredients | INCI | Blue, % |
|---|---|---|
| R4223-29 (Ex. 18) | Suncroma Ultramarine Blue | 8 |
| Dub 810 C | Cococaprylate/caprate | 27 |
| Premium Organic Olive Butter | - | 10 |
| C86-6105 Satin Mica | Mica | 30 |
| C83-1254 Sunpuro Deep Blue | Mica & Titanium dioxide & Iron Blue | 5 |
| C81-1258 Sunpuro Iridescent Blue | Mica and Titanium Dioxide | 10 |
| C81-3760 SunShine Super Glitter Green | Mica and Titanium Dioxide | 5 |
| Sepifine BB | Amylopectin | 5 |
| Isododecane | Isododecane | +2.32g |

**Table 22 - 2-in-1 Cream-to-Powder Green Pearly Eye Shadow (Example 55) (% by weight)**

| Ingredients | INCI | Green, % |
|---|---|---|
| R4223-30 (Ex. 19) | Suncroma Chromium oxide | 4 |
| R4223-18 (Ex. 17) | Sunpuro Titanium dioxide | 4 |
| Dub 810 C | Cococaprylate/caprate | 27 |
| Premium Organic Olive Butter | - | 10 |
| C86-6105 Satin Mica | Mica | 25 |
| C91-4161 Intenza Envy | FD&C Blue 1 Al Lake & FD&C Yellow 5 Al Lake | 25 |
| Sepifine BB | Amylopectin | 5 |
| Isododecane | Isododecane | +4.6g |

**Table 23 - 2-in-1 Cream-to-Powder Violet Pearly Eye Shadow (Example 56) (% by weight)**

| Ingredients | INCI | Violet, % |
|---|---|---|
| R4223-31 (Ex. 20) | Suncroma Manganese violet | 8 |
| Dub 810 C | Cococaprylate/caprate | 27 |
| Premium Organic Olive Butter | - | 10 |
| C86-6105 Satin Mica | Mica | 22 |
| Sunshine royal violet C83-3240 | Mica and Titanium Dioxide and Red Iron Oxide and Carmine | 5 |
| SunShine violet aurora C92-4240 | Mica and Titanium Dioxide | 23 |
| Sepifine BB | Amylopectin | 5 |
| Isododecane | Isododecane | +1.9g |

**Table 24 - Procedure for all Cream-Powder Applications**

| |
|---|
| 1. Heat all the dry dispersions in the color composition in a beaker |
| 2. When all the dispersions are properly melted, add oils and butters, hand mix well |
| 3. Weigh all the powders in another beaker and pour them slowly in the melt mixture while hand mixing and heating gently. Mix until there are no more lumps. |
| 5. If the mixture is too dry, add solvent drop by drop. |
| 6. Allow time to dry and solidify |

**Table 25 - W/O Emulsion CC Cream (Example 57)(% by weight)**

| Phase | Ingredients | INCI | % |
|---|---|---|---|
| A | Water | Water | 52.56 |
| | Sodium Chloride | Sodium Chloride | 1.15 |
| | Glycerin | Glycerin | 2.3 |
| B | Bentone Gel Abo V | Crambe Abyssinica Seed Oil, Stearalkoium Hectorite, Propylene Carbinate | 7.41 |
| | Imwittor 600 | Polyglyceryl-3 Polyricinoleate | 3.45 |
| | Uvinul^{®} MC 80 | Ethylhexyl Methoxycinnamate | 8.62 |
| | Vitamin E Acetate USP | Tocopheryl Acetate | 0.12 |
| | Multiple Hydration Complex | Carthamus Tinctorius (Safflower) Seed Oil, Limnanthes Alba (Meadowfoam) Seed Oil, Olea Eurpaea (Olive) Oil Unsaponifiables, Lecithin, Butyrospremum Parkii (Shea) Butter | 3.45 |
| | Dub ININ | Isononyl Isononanoate | 5.17 |
| | CERAPHYL^{®} 375 | Isostearyl Neopentanoate | 5.17 |
| C | R4223 - 18 (Ex. 17) | Titanium Dioxide Dry Dispersion | 4.3 |
| | R4223 - 53 (Ex. 7) | Yellow Iron Oxide Dry Dispersion | 1 |
| | R4223 - 52 (Ex. 8) | Red Iron Oxide Dry Dispersion | 0.5 |
| | R4223 - 54 (Ex. 9) | Black Iron Oxide Dry Dispersion | 0.2 |
| D | Euxyl K701 | Phenoxyethanol, Benzoic Acid, Dehydroacetic Acid, Ethylhexylglycerin | 1.15 |
| | Gatuline Expression | Alcohol & Water & Acmella Oleracea Extract | 1.15 |
| | Vegetol Aloe ME200 Hydro | Water & Propylene Glycol & Aloe Ferox Leaf Extract | 1.15 |
| | Vegetal Cp GR049 Hydro | Propylene Glycol & Cucumis Sativus Fruit Extract | 1.15 |
| Procedure | 1. Premix Part A, B & C separately and heat each to 80-85°C | | |
| | 2. When C is melted and homogeneous, mix together with B | | |
| | 3. Add Part A to B/C with a Rayneri for 10 minutes, to form the emulsion | | |
| | 4. Cool to below 40°C, then add part D ingredients to the batch and mix well | | |
| | 5. Fill into appropriate container. | | |

**Table 26 - W/O Emulsion BB Cream (Example 58) (% by weight)**

| Phase | Ingredients | INCI | % |
|---|---|---|---|
| A1 | KSG-210 | Dimethicone & Dimethicone/PEG-10/15 Crosspolymer | 5 |
| | KSG-16 | Dmethicone & Dimethicone/Vinyl Dimethicone Crosspolymer | 3 |
| | KF-6038 | Lauryl PEG-9 Polydimethylsiloxyethyl Dimethicone | 3 |
| | Bentone Gel NGD-V | Neopenthyl Glycol Diheptanoate & Disteardimonium Hectorite & Propylene Carbonate | 6.7 |
| | Crodamol TN | Isotridecyl Isononanoate | 1.5 |
| | KF-96A-6cs | Dimethicone | 8 |
| | KF-56A | Diphenylsiloxy Phenyl Trimethicone | 8 |
| | Uvinul MC 80 | Ethylhexyl methoxycinnamate | 5 |
| A2 | KSP-100 | Vinyl Dimethicone/Methicone Silsesquioxane Crosspolymer | 3 |
| B1 | Water | Water | 41.1 |
| | Trisodium Citrate | Sodium Citrate | 0.2 |
| | Sodium Chloride | Sodium Chloride | 0.5 |
| B2 | Butylene Glycol | Butylene Glycol | 5 |
| C | R4223 - 18 (Ex. 17) | Titanium Dioxide Dry Dispersion | 4.3 |
| | R4223 - 53 (Ex. 7) | Yellow Iron Oxide Dry Dispersion | 1 |
| | R4223 - 52 (Ex. 8) | Red Iron Oxide Dry Dispersion | 0.5 |
| | R4223 - 54 (Ex. 9) | Black Iron Oxide Dry Dispersion | 0.2 |
| | | | 96 |
| Procedure | 1. Mix A1 ingredients with a disperser until homogeneous. | | |
| | 2. Add A2 to Al phase, then disperse | | |
| | 3. Mix B1 then add B2 under stirring | | |
| | 4. Emulsify by adding B to A under low shearing (700 to 1000 rpm). Then, increase speed up to 3000 rpm. | | |
| | 5. Melt phase C until homogeneous | | |
| | 6. Add slowly A/B to C while heating constantly and mixing | | |

**Table 27 - O/W Face Primer (Example 59) (% by weight)**

| Phase | Ingredients | INCI | % |
|---|---|---|---|
| A | Water | Water | 62.85 |
| | Carbopol Ultrez 10 | Carbomer | 0.1 |
| | Propylene Glycol | Propylene Glycol | 5 |
| | Pemulen TR-1 | Acrylates/C10-30 Alkyl Acrylate Crosspolymer | 0.1 |
| B | 6290 Berry Wax | Rhus Verniciflua Peel Cera | 10 |
| | Jojoba Oil Colorless CP RBD | Simmondsia Chinesis Seed Oil | 5 |
| | Blanova Apricot Kernel Oil Refined | Apricot Kernel Oil | 5 |
| | Tocomix L50 IP | Tocopherol | 0.2 |
| | Montanov S | Coco-Glucoside & Coconut Alcohol | 3 |
| | LexFeel natural N50 MB | Diheptyl Succinate (and) Capryloyl Glycerin/Sebacic Acid Copolymer | 1.25 |
| C | R4223 - 18 (Ex. 17) | Titanium Dioxide Dry Dispersion | 4.3 |
| | R4223 - 53 (Ex. 7) | Yellow Iron Oxide Dry Dispersion | 1 |
| | R4223 - 52 (Ex. 8) | Red Iron Oxide Dry Dispersion | 0.5 |
| | R4223 - 54 (Ex. 9) | Black Iron Oxide Dry Dispersion | 0.2 |
| D | NaOH (12.5%) | Aqua, Sodium Hydroxide | 0.5 |
| | Euxyl PE 9010 | Phenoxyethanol (and) Ethylhexylglycerin | 1 |
| Procedure | 1. Blend water & Carbomer without heating or agitation. Wait for 10 minutes. | | |
| | 2. When the carbomer has sedimented, add first Propylene Glycol then, under stirring, Pemulen TR-1. | | |
| | 3. Heat separately phase B & C until melted; when homogeneous, slowly add phase B to C | | |
| | 4. Heat phase A (under stirring) and phase B/C separately to 80°C. | | |
| | 5. Add Part B to A under agitation, to form the emulsion | | |
| | 6. Add the NaOH under stirring at 60°C; mix for 1 to 3 minutes. | | |
| | 7. Cool to below 40°C, then add preservative to the batch and mix well | | |
| | 8. Fill into appropriate container. | | |

**Table 28 - Solid Bar Soap or Solid Shampoo bar (Example 60) (% by weight)**

| Ingredients | INCI | % |
|---|---|---|
| R4223-29 (Ex. 18) | CI 77007 Ultramarine Blue | 0.12 |
| R4223-18 (Ex. 17) | CI 77891 Titanium Dioxide | 0.18 |
| SCI | Sodium Cocoyl Isethionate | 60.5 |
| C86-3222 SynMica Super | Synthetic Fluorphlogopite | 3.2 |
| Olivem 1000 | Cetearyl Olivate & Sorbitan Olivate | 12 |
| Almond oil | Almond oil | 12 |
| Coconut Oil | Coconut Oil | 12 |

Melt and disperse dry dispersions in oil first and add to the rest of the ingredients.

**Table 29 - Sweet Almond Solid bar (Example 61) (% by weight)**

| Ingredients | INCI | % |
|---|---|---|
| R4223-52 | CI 77491 Red Iron Oxide | 0.02 |
| R4223-18 | CI 77891 Titanium Dioxide | 0.03 |
| Olive oil | Olive Oil | 51.55 |
| Almond oil | Almond oil | 3.4 |
| Coconut Oil | Coconut Oil | 13.6 |
| Caustic soda | Sodium Hydroxide | 9 |
| Sweet Almond Milk | Prunus Dulcis (Sweet Almond Milk) | 8.1 |
| Water | Water | 14.3 |

Melt and disperse dry dispersions in oil first and add to the rest of the ingredients.

To demonstrate the low-dusting properties a test was performed using the following protocol:
10g of each sample under test was poured into a clean dry 500 ml graduated cylinder.

Results were quantified by observing the ml the dust traveled up the cylinder after pouring and recorded in Table 3.

The further the dust traveled up the cylinder, the more prone the example is to dusting.

**Table 30: Dusting Data for Examples 1, 4, 6 dispersions vs. comparative pigments ("Distance Dust Traveled" unit is volume)**

| Example | Distance Dust Traveled (ml) |
|---|---|
| R4223-59 (Ex. 1 dispersion) | 150 |
| R4223-28 Ex. 4 dispersion) | 250 |
| R4223-27 (Ex. 6 dispersion) | 150 |
| FD&C Yellow 5 Al lake (comparative pigment) | 500 + |
| D&C Red 7 Ca lake (comparative pigment) | 500 + |
| D&C Red 28 Al Lake (comparative pigment) | 500 + |

The data in Table 30 clearly exhibits the lower dusting of the inventive dispersions vs. comparative pigments.

## Claims

1. A solid pigment dispersion comprising one or more pigments approved for cosmetic use and a combination of one or more oils and one or more waxes that are plant-based, wherein the dispersion is in the form of a dry powder, wherein the pigment is present in an amount from 25-75% by weight, and the ratio of wax:oil is from 1:1 to 4:1, and wherein the particle size of the pigment is less than 15 µm as measured on a Hegman grind gauge according to ASTM D1210-05(2014) with additional heat, wherein the method involves the application of the pre-melted dispersion on the Hegman gauge 50-0 microns channel, pre-heated to 50 °C, using a scraper in order to obtain a continuous layer in the well from top to bottom.

2. The dispersion of claim 1, wherein all of the oils and/or waxes are plant-based.

3. The dispersion of claim 1, wherein the dispersion has a melting point greater than 30°C.

4. The dispersion of claim 1, wherein the dispersion has a melting point greater than 50°C.

5. The dispersion of claim 1, wherein the particle size of the pigment is less than 10 µm.

6. The dispersion of claim 1, wherein the wax is selected from the group consisting of rapeseed, soybean, sunflower, olive, castor bean, coconut, canola, jojoba, argan, pistachio, apricot kernel, sumac wax and combinations thereof.

7. The dispersion of claim 1, wherein the oil is selected from the group consisting of heptyl undecylenate, coco-caprylate, coco-caprylate/caprate, hydrogenated ethylhexyl olivate/hydrogenated olive oil unsaponifiables, propanediol dicaprylate, olive oil decyl esters/squalene and combinations thereof.

8. A composition comprising the dispersion of any one of claims 1-7.

9. The composition of claim 8, wherein the composition is a personal care product or cosmetic formulation.

10. A method for providing a solid pigment dispersion according to claim 1, comprising grinding a pigment that meets regulatory guidelines for cosmetic applications, to a particle size less than 15 µm, into a medium comprised of one or more oils and one or more waxes that are plant-derived, wherein the dispersion is in a powder form.

11. The method of claim 10, wherein the particle size is less than 10 µm.

## Patentansprüche

1. Feste Pigmentdispersion, umfassend ein oder mehrere Pigmente, die für die kosmetische Verwendung zugelassen sind, und eine Kombination von einem oder mehreren Ölen und einem oder mehreren Wachsen, die auf pflanzlicher Basis sind, wobei die Dispersion in Form eines trockenen Pulvers vorliegt, wobei das Pigment in einer Menge von 25 bis 75 Gew.% vorliegt und das Verhältnis von Wachs:Öl 1:1 bis 4:1 beträgt, und wobei die Partikelgröße des Pigments kleiner als 15 µm ist, gemessen auf einem Hegman Mahlfeinheitsmessgerät gemäß ASTM D1210-05(2014) mit zusätzlicher Wärme, wobei das Verfahren das Aufbringen der vorgeschmolzenen Dispersion auf den 50-0 Mikrometer-Kanal des Hegman Messgeräts, der auf 50 °C vorgewärmt ist, unter Verwendung eines Schabers beinhaltet, um in der Mulde eine kontinuierliche Schicht von oben nach unten zu erhalten.

2. Dispersion nach Anspruch 1, wobei alle der Öle und/oder Wachse auf pflanzlicher Basis sind.

3. Dispersion nach Anspruch 1, wobei die Dispersion einen Schmelzpunkt höher als 30 °C hat.

4. Dispersion nach Anspruch 1, wobei die Dispersion einen Schmelzpunkt höher als 50 °C hat.

5. Verfahren nach Anspruch 1, wobei die Partikelgröße des Pigments kleiner als 10 µm ist.

6. Dispersion nach Anspruch 1, wobei das Wachs ausgewählt ist aus der Gruppe bestehend aus Rapssaat, Soja, Sonnenblume, Olive, Rizinus, Kokosnuss, Canola, Jojoba, Argan, Pistazie, Aprikosenkern, Sumachwachs und Kombinationen davon.

7. Dispersion nach Anspruch 1, wobei das Öl ausgewählt ist aus der Gruppe bestehend aus Heptylundecylenat, Cococaprylat, Coco-caprylat/-caprat, hydriertem Ethylhexylolivat/hydrierten unverseifbaren Olivenölbestandteilen, Propandioldicaprylat, Olivenöl-Decylestern/Squalen und Kombinationen davon.

8. Zusammensetzung, umfassend die Dispersion nach einem der Ansprüche 1 bis 7.

9. Zusammensetzung nach Anspruch 8, wobei die Zusammensetzung ein Körperhygieneprodukt oder eine Kosmetikformulierung ist.

10. Verfahren zum Bereitstellen einer festen Pigmentdispersion nach Anspruch 1, umfassend Mahlen eines Pigments, das die regulatorischen Richtlinien für Kosmetikanwendungen erfüllt, auf eine Partikelgröße von weniger als 15 µm, in ein Medium hinein, das aus einem oder mehreren Ölen und einem oder mehreren Wachsen zusammengesetzt ist, die von Pflanzen abgeleitet sind, wobei die Dispersion in einer Pulverform vorliegt.

11. Verfahren nach Anspruch 10, wobei die Partikelgröße kleiner als 10 µm ist.

## Revendications

1. Dispersion de pigment solide comprenant un ou plusieurs pigments approuvés pour une utilisation cosmétique et une combinaison d'une ou de plusieurs huiles et d'une ou de plusieurs cires qui sont à base végétale, la dispersion étant sous la forme d'une poudre sèche, le pigment étant présent en une quantité de 25 à 75 % en poids, et le rapport de cire : huile étant de 1 : 1 à 4 : 1, et la taille de particule du pigment étant inférieure à 15 µm telle que mesurée sur une jauge de broyage d'Hegman selon la norme ASTM D1210-05(2014) avec de la chaleur supplémentaire, le procédé impliquant l'application de la dispersion pré-fondue sur le canal 50-0 microns de la jauge d'Hegman, pré-chauffée à 50 °C, en utilisant un racloir afin d'obtenir une couche continue dans le puits du haut vers le bas.

2. Dispersion selon la revendication 1, toutes les huiles et/ou cires étant à base végétale.

3. Dispersion selon la revendication 1, la dispersion ayant un point de fusion supérieur à 30 °C.

4. Dispersion selon la revendication 1, la dispersion ayant un point de fusion supérieur à 50 °C.

5. Dispersion selon la revendication 1, la taille de particule du pigment étant inférieure à 10 µm.

6. Dispersion selon la revendication 1, la cire étant choisie dans le groupe constitué par une cire de colza, de soja, de tournesol, d'olive, de graine de ricin, de noix de coco, de canola, de jojoba, d'argan, de pistache, de noyau d'abricot, de sumac et des combinaisons correspondantes.

7. Dispersion selon la revendication 1, l'huile étant choisie dans le groupe constitué par l'undécylénate d'heptyle, le caprylate de coco, le caprylate/caprate de coco, l'olivate d'éthylhexyle hydrogéné/les insaponifiables d'huile d'olive hydrogénée, le dicaprylate de propanediol, les esters décyliques d'huile d'olive/squalène et des combinaisons correspondantes.

8. Composition comprenant la dispersion selon l'une quelconque des revendications 1 à 7.

9. Composition selon la revendication 8, la composition étant un produit de soin personnel ou une formulation cosmétique.

10. Procédé pour fournir une dispersion de pigment solide selon la revendication 1, comprenant le broyage d'un pigment qui satisfait les directives règlementaires pour des applications cosmétiques, jusqu'à une taille de particule inférieure à 15 µm, dans un milieu composé d'une ou de plusieurs huiles et d'une ou de plusieurs cires qui sont à base végétale, la dispersion étant sous une forme de poudre.

11. Procédé selon la revendication 10, la taille de particule étant inférieure à 10 µm.
